# EUROPEAN PATENT APPLICATION

(11) **EP 2 505 208 A1**
(43) Date of publication of application: **03.10.2012**
(21) Application number: 11160817.0
(22) Date of filing: 01.04.2011
(51) Int. Cl.: A61K 39/102

(54) **Vaccine against Pasteurellaceae**

(71) Applicant: University of Graz, 8010 Graz (AT)
(72) Inventor: Schild, Stefan, Dr. rer. nat., 8042 Graz (AT); Roier, Sandro, 8940 Weißenbach bei Liezen (AT); Reidl,Joachim, Dr. rer. nat., 8010 Graz (AT)
(74) Representative: Patentanwaltskanzlei Matschnig & Forsthuber OG

(57) **Abstract**

The invention relates to vaccines providing protection against infections caused by members of the *Pasteurellaceae* family comprising outer membrane vesicles as the only active components, wherein the outer membrane vesicles are obtained from one or more strains of the *Pasteurellaceae* family, with the proviso that hypervesiculating strains are excluded. The invention also relates to a method for preparing such a vaccine.

## Description

The present invention relates to vaccines providing protection against infections caused by members of the *Pasteurellaceae* family comprising outer membrane vesicles obtained from *Pasteurellaceae* strains.

The *Pasteurellaceae* family comprises a large number of Gram-negative proteobacteria including commensals as well as human and animal pathogens. The most important genera are *Haemophilus, Actinobacillus, Pasteurella,* and *Mannheimia.*

*Actinobacillus, Pasteurella,* and *Mannheimia* species are primarily animal pathogens. For example, the closely related *Pasteurella multocida* and *Mannheimia haemolytica* (formerly *Pasteurella haemolytica* biotype A) are frequently implicated in bovine respiratory disease (BRD). Generally these bacteria are commensals in the nasopharynx of many domestic and wild animals including cattle. However, they can establish severe lung infections in animals that are subjected to stress (e.g. transportation, weaning, overcrowding). BRD is one of the most important health problems faced by beef and dairy producers causing death losses, higher medication and labour costs, reduced carcass value and loss of production. BRD may cost the U.S. cattle industry over 500 million US$ each year. In Austria an outbreak of avian cholera (fowl cholera, avian pasteurellosis) caused by *P. multocida* in a chicken farm resulted in 6800 infected animals two years ago. In humans the major type of infection caused by *Pasteurella,* and *Mannheimia* are wound infections due to bits and scratches from animals like dogs, cats, and horses. Respiratory tract and invasive infections are less common, but can lead to severe complications in infants and immunocompromised patients.

Within the *Pasteurellaceae* family the genus *Haemophilus* comprises probably the most important human pathogens. For example the sexually transmitted *Haemophilus ducreyi* causing genital ulcers and *Haemophilus influenzae* causing pneumonia, meningitis, sepsis, otitis media, sinusitis, adult epiglottitis, and obstetrical infections. Isolates of *H. influenzae* can be divided into encapsulated and unencapsulated strains, referred to as nontypeable strains (NTHi), according to the presence of a polysaccharide capsule. The capsule is the major virulence factor of invasive strains. Encapsulated strains belong to the serotypes a to f with b being the most virulent one. Since the introduction of an effective vaccine for *H. influenzae* type b (Hib) the focus has now turned to NTHi. Although nasopharyngeal colonization of NTHi can be asymptomatic, they are frequently associated with otitis media, chronic bronchitis, community-acquired pneumonia, and obstetrical infections. NTHi is the most common cause of exacerbations of chronic obstructive pulmonary disease (COPD) as well as bronchiectasis and also causes infections in people with cystic fibrosis. According to the latest estimations by the WHO in 2007, currently 210 million people have COPD and 3 million people died of COPD in 2005. Furthermore COPD is projected to be the third most common cause of death and fifth most common cause of disability in the world by 2030.

Effective vaccines to prevent infections caused by most members of the *Pasteurellaceae* are currently lacking. In the case of BRD *M. haemolytica* and *P. multocida* immunizing products are commercially available, but not all vaccines have consistently shown benefits in feedlot programs. The pneumoccocal polysaccharide protein D-conjugated vaccine "Synflorix" uses the protein D of NTHi strains as a carrier for various pneumococcal polysaccharides. Thus, vaccination with Synflorix induces a certain level of antibodies against protein D. However, the focus of this vaccine is protection against pneumococcal infections and the efficacy rate against NTHi is only around 30%. Currently many new reformulated products targeting causative agents of BRD and NTHi-induced infections are under investigation demonstrating the need of new effective and cheap vaccine candidates. One noticeable exception is the capsule polysaccharide conjugate vaccine against human infections caused by Hib. Since the introduction of the Hib vaccine in the early 90s Hib infections have markedly decreased, whereas infections caused by NTHi have increased. One challenge in developing a vaccine against NTHi is the absence of a capsule, which is the basis of the Hib vaccine. The Hib-Vaccine does not confer protection against NTHi. Furthermore, NTHi strains show significant heterogeneity in outer membrane (OM) protein patterns and other proteins have highly variable regions. Several OM components of NTHi have been proposed as potential vaccine candidates.

OMVs, also referred to as "blebs", are small membrane spheres having a diameter of approximately 10 to 300 nm. OMVs are naturally released from the outer membrane of Gram-negative bacteria during growth. OMVs accumulate in the culture medium when bacteria are grown in the laboratory, and can be purified and stored. OMVs are non-living and contain a number of important protective antigens such as outer membrane proteins, periplasmic proteins, lipids, and the lipooligosaccharides (LOS). For example, the classification of different NTHi-serotypes is based on their respective LOS-structure.

Purified OMVs of some pathogens have been demonstrated as useful in vaccine development and in antibiotic therapy. Vaccines based on outer membrane vesicles (OMVs) are known in the art. Upscale to industrial production has already been established for OMVs from *Neisseria meningitidis,* which have been extensively studied and tested in a limited manner as vaccines to contain outbreaks of Serogroup B *N. meningitidis* meningitis (Girard et al., 2006, Vaccine 24:4692-4700).

WO 2009/049013 describes an OMV-based vaccine composition against *Vibrio cholerae* and, in some embodiments, other pathogens that cause diarrhea.

US 2002/0028215 A1 discloses a vaccine comprising membrane vesicles derived from a pathogen, wherein the membrane vesicles are integrated into the cell surface of a carrier strain. The pathogen may be a member of the *Pasteurellaceae* family including *H. influenzae, M. haemolytica* and *P. multocida.* Experimental data are only shown for membrane vesicles obtained from *Shigella* and *Pseudomonas* strains. The object of US 2002/0028215 A1 is to enhance immunogenicity and achieve a protein-induced immune response by fusing the membrane vesicles to the carrier strain. Generally, this is desired for most vaccines directed against Gram-negative bacteria, since a polysaccharide-induced immune response, mainly against LPS (Lipopolysaccharide), does not result in cross-protection against intra-species variants and serogroups, respectively. However, the fusion of membrane vesicles to a carrier strain is disadvantageous with respect to a cost-efficient and convenient vaccine production, since either attenuated or inactivated carrier strains have to be used.

US 2004/0116665 A1 relates to Gram-negative bacterial strains that are genetically engineered to "hyperbleb" by down-regulating expression of one or more *tol* genes or attenuating the peptidoglycan-binding activity by mutation of one or more gene(s) encoding a protein comprising a peptidoglycan-associated site. "Hyperblebbing" strains (also referred to as "hypervesiculating" strains) release increased levels of OMVs. Among others, US 2004/0116665 A1 also refers to *H. influenzae* strains including NTHi. According to US 2004/0116665 A1 it is difficult to obtain or prepare effective, consistent outer membrane vesicle preparations from strains that do not hyperbleb (hypervesiculate). Scientific data from the literature, however, demonstrate that hyperblebbing is a result of disordered outer membrane integrity. Tol/Pal and P5/OmpA mutations as taught in US 2004/0116665 A1 are reported to cause general membrane instability, differences in the protein-content as well as structural impairment of the outer membrane and the OMVs (Kulp and Kuehn, 2010, Rev Microbiol 64: 163-84; Lazzaroni et al., 1999, FEMS Microbiol Lett 177:191-7; Llamas et al., 2000, J Bacteriol 182:4767-72; Deatherage et al., 2009, Mol Microbiol 72:1395-405; McBroom et al., 2006, J Bacteriol 188:5385-92; Song et al., 2008, Mol Microbiol 70:100-11; Serino et al., 2007, Mol Microbiol 64:1391-403; Sonntag et al., 1978, J Bacteriol 136:280-5). The use of hyperblebbing (hypervesiculating) mutants leads to an increase of undesirable products in OMVs. Moreover, hyperblebbing may lead to incorrect protein folding, thereby not presenting the antigens in their native conformation. Furthermore, the outer membrane protein A (OmpA) is important for the serum resistance and pathogenicity (Weiser and Gotschlich, 1991, Infect Immun 59:2252-8). Consequently, vaccines inducing an immune response against OmpA are advantageous, which is not the case with OmpA-mutants as taught by US 2004/0116665 A1.

It is an object of the present invention to meet the high demand for a vaccine providing protection against infections caused by members of the *Pasteurellaceae* family in humans and animals.

It is a further object of the invention to provide an effective, low-priced and broad-spectrum vaccine against *Pasteurellaceae* infections, which does not have the above-discussed disadvantages of known *Pasteurellaceae* vaccines. Specifically, an effective and broad-spectrum vaccine against NTHi should be provided.

This object is achieved by a vaccine comprising outer membrane vesicles as the only active components, wherein the outer membrane vesicles are obtained from one or more strains of the *Pasteurellaceae* family, with the proviso that hypervesiculating strains are excluded.

Surprisingly, it has been found that the vaccine according to the invention induces a robust immune response as well as an unexpected and significant cross-protection against other members of the *Pasteurellaceae* family. The enhanced cross-protection may be explained by the presence of conserved outer membrane antigens. A cross-protection to this extent has not been observed for any vaccine against *Pasteurellaceae* so far.

US 2002/0028215 implicates that the use of membrane vesicles without a carrier strain results in a polysaccharide-induced immune response. Therefore, a vaccine comprising OMVs as the only active components is neither desired nor disclosed or indicated in this document. US 2002/0028215 teaches away from a vaccine according to the present invention. In the vaccine according to the invention OMVs represent the only active components, wherein the term "only active components" as used herein is a term used to indicate that the OMVs are the only components in the vaccine capable of inducing an immunogenic response in a subject.

Hypervesiculating (hyperblebbing) strains such as those disclosed in US 2004/0116665 A1 are explicitly excluded due to the well-known disadvantages as discussed above. Therefore, the terms "hyperblebbing strains" and "hypervesiculating strains" as used herein interchangeably refer to Gram-negative strains of the *Pasteurellaceae* family with increased vesicle shedding properties, i.e. shedding an increased quantity of outer membrane vesicles per bacterial cell in comparison to the quantity of an unmodified strain. This exclusion refers to all kinds of hypervesiculating strains, e.g. strains in which hypervesiculation is induced by defined mutagenesis or random mutagenesis with the aim to create hypervesiculating strains.

Vaccines according to the present invention have the following advantages:
- OMVs derived from *Pasteurellaceae* can be isolated by simple and established purification steps.
- Isolated OMVs derived from *Pasteurellaceae* are stable (even at 37°C). Thus, a cold-chain is not required for shipping and administration of a *Pasteurellaceae* vaccine according to the invention.
- Non-invasive intranasal or alternatively oral immunization routes are possible, wherein no adjuvant is needed. OMVs are non-living and non-propagating particles. Thus, there is a low risk for severe side-effects known for live-attenuated vaccine candidates and the productions costs can be kept down.
- OMVs are naturally released and therefore represent the surface structure of a bacterial outer membrane in its native conformation. This is a huge advantage compared to vaccine candidates based on purified proteins of the outer membrane. Vaccines based on such purified proteins are usually very expensive due to the high productions costs. Additionally, the proteins can denaturate during the purification steps. Thus, the purified proteins present in the final vaccine are likely to have an altered conformation and different antigenic profile in comparison to the native situation. This problem is overcome by the present invention.
- The inventors could demonstrate that OMVs from *Pasteurellaceae* are highly immunogenic and induce a robust protective immune response without adjuvance as it will become evident by the results presented in the examples below.
- Vaccines according to the invention were found to provide significant cross-protection against other members of the *Pasteurellaceae* family. Purified OMVs derived from several strains of the *Pasteurellaceae* family can be mixed to obtain a complex multifarious immune response. Such OMV-mixtures can be assorted in suitable ratios to deliver a broad spectrum of antigens that reflect the heterogeneity of the strains. Such mixtures induce a complex immune response against multiple different antigens resulting in a significantly enhanced cross-protection as demonstrated in the examples disclosed herein.

The efficacy of the novel *Pasteurellaceae* vaccines according to the invention is demonstrated in mice with immunization mixes comprising OMVs derived from heterogenous NTHi strains and *P. multocida* as representatives of the whole *Pasteurellaceae* family (see examples below). The results presented in the examples indicate great potential for a flexible and broad-spectrum vaccine directed against a variety of members within the *Pasteurellaceae* family depending on the OMVs combined in the immunization mix, for example, combining OMVs derived from heterogenous NTHi strains to obtain a vaccine against NTHi infections or OMVs derived from *P. multocida* to obtain a vaccine against common causes of bovine respiratory disease (BRD) and avian cholera.

The vaccine, which is preferably an immunogenic composition comprising the OMVs and a pharmaceutically acceptable diluent and/ or carrier, is able to raise an immune response in a patient, wherein the immune response is directed against the antigens present in the OMVs.

In one aspect, the vaccine comprises outer membrane vesicles obtained from one *Pasteurellaceae* strain only.

In another aspect, the vaccine comprises outer membrane vesicles obtained from more than one strain of the *Pasteurellaceae* family. Purified OMVs derived from several strains of the *Pasteurellaceae* family can be mixed. Such OMV-mixtures can be assorted in suitable ratios to deliver a broad spectrum of antigens that reflect the heterogeneity of the strains. Such mixtures induce a complex immune response against multiple different antigens resulting in a significantly enhanced cross-protection.

The term "suitable ratio" as used herein refers to generally mixed equally on the basis of the protein amount of the respective OMV samples or mixtures of other appropriate ratios to obtain the most effective immune response. The suitable ratio may be determined by means of methods known by those skilled in the art, e.g. routine trials.

In yet another aspect, the vaccine according to the invention may comprise outer membrane vesicles obtained from one or more strains selected from the group consisting of wildtype strains, genetically modified strains, and combinations thereof. In other words, the vaccine may comprise OMVs obtained from (i) one wildtype strain or (ii) one genetically modified strain, or it may comprise a mixture of OMVs obtained from (iii) two or more wildtype strains, (vi) two or more genetically modified strains or (v) at least one of a wildtype and at least one of a genetically modified strain.

As used herein, the term "wildtype strain" refers to a given *Pasteurellaceae* strain that has the genotypic or phenotypic characteristics of a naturally occurring strain, i.e. a non-mutant strain, isolated from a naturally occurring source, e.g. isolated from a subject infected with this strain.

In contrast, the terms "genetically modified", "mutant" or "recombinant" as interchangeably used herein refer to a given *Pasteurellaceae* strain having modifications in its genetic pattern as a result of mutation and displaying altered characteristics and/or an altered phenotype when compared to the wildtype strain. Strains may be genetically modified, e.g., in a targeted manner by genetic engineering techniques, or randomly by induced mutation. Furthermore, the term "genetically modified" also refers to naturally occurring mutants which can be isolated from a natural source and identified by their altered characteristics in comparison to their respective wildtype strains. Examples for naturally occurring mutant strains are spontaneous antibioticresistant strains, strains having an altered stability or pathogenicity as well as phase variants altering capsule expression or composition and amino acid sequence of outer membrane proteins or composition and expression of polysaccharide structures. Additionally, mutants (e.g. *tfox)* with higher natural competence occur.

The following, non-restrictive examples of genetically modified *Pasteurellaceae* strains are encompassed by the present invention:
- Recombinant strains bearing a selective marker enabling monitoring, detection, selection and counter-selection, e.g., antibiotic-resistance cassettes, heavy-metal resistance, sucrose sensitivity, temperature sensitivity, auxotrophic markers *etc.*
- Recombinant strains having an increased fitness *per se* or with respect to the OMVs isolated therefrom, e.g. enhanced stability, increased lifespan *etc.* For example, strains expressing NadV allowing growth on nicotinamide as sole source of factor V or sxy/ *tfox*-mutations resulting in enhanced growth rates (Sauer et al., 2004, Antimicrob Agents Chemother 48:4532-4541; Redfield et al., 1991, J Bacteriol 173:5612-5618).
- Recombinant strains no longer having disadvantageous structures, e.g. bacterial toxins. For example, mutants lacking Pasteurella toxin, mutants reducing lipid_A toxicity, capsule mutants to facilitate detection of antigens located near the outer membrane surface by the immune system. (Labandeira-Rey et al., 2010, Infect Immun 78:4779-4791; Wilson et al., 2010, Future Microbiol 5:1185-1201; Lee et al., 2009, J Microbiol Biotechnol 19:1271-1279).

The outer membrane's structure (and, consequently, the outer membrane vesicles's structure) of a given recombinant strain is preferably not significantly, more preferably not at all, altered when compared to the wildtype strain's outer membrane structure and wildtype OMVs, respectively. Therefore, OMVs displaying a wildtype structure are particularly preferred, because they best represent the surface structure of a native bacterial outer membrane conformation providing an antigenic profile reflecting the natural situation.

Again, it is explicitly emphasized that wildtype or genetically modified strains having a hypervesiculating (hyperblebbing) phenotype as defined and discussed above are excluded because of the aforementioned reasons.

A vaccine according to the invention is suitable for human and/ or veterinary use.

The terms "patient" and "subject" as used herein interchangeably refer to humans or animals. In many aspects, the subject is a human being. For example, within the *Pasteurellaceae* family, the genus *Haemophilus* comprises probably the most important human pathogens such as typeable and nontypeable *H. influenzae* and *H. ducreyi.* In other aspects, the subjects are farm animals or domestic animals such as cattle, horses, poultry, cats or dogs. However, the vaccine may also be applied to wild animals. For example, the genera *Actinobacillus, Pasteurella,* and *Mannheimia* are primarily animal pathogens. In humans the major type of infection caused by *Pasteurella,* and *Mannheimia* are wound infections due to bits and scratches from animals like dogs, cats, and horses.

Vaccines according to the invention may either be prophylactic in order to prevent *Pasteurellaceae* infections or therapeutic in order to treat a condition after infection. Typically the vaccine is intended for prophylactic treatment, i.e. the induced immune response is preferably protective.

Vaccines according to the invention are, therefore, useful in treating or preventing infections and diseases caused by members of the *Pasteurellaceae* family, typically pathogenic members, in particular strains of the genera *Haemophilus, Actinobacillus, Pasteurella,* and *Mannheimia.* The current data demonstrate a robust immune response and enhanced cross-protection against other members within the *Pasteurellaceae* family not present in the vaccine.

In certain embodiments the vaccine comprises outer membrane vesicles obtained from one or more *H. influenzae* strains. As already explained above, *H. influenzae* isolates can be divided into encapsulated and unencapsulated strains, according to the presence of a polysaccharide capsule. Unencapsulated strains are referred to as nontypeable strains (NTHi). Therefore, the term *"H. influenzae"* as used herein refers to both encapsulated and unencapsulated strains. In certain preferred embodiments, the one or more *H. influenzae* strains are NTHi. Non-exhaustive examples are sequenced strains (e.g. NTHi 86-028NP), clinical isolates that are comprehensively characterized and demonstrate high heterogeneity with respect to their surface composition (e.g. NTHi strains 9274, 7502, 5657, 3198, 2019, 1479) and strains capable to cause invasive disease (Harrison et al., 2005, J Bacteriol 187:4627-4636; Gu et al., 1996, Infect Immun 64:4047-4053; Murphy et al., 1985, Infect Immun 50:15-21; Erwin et al., 2005, Infect Immun 73:5853-5863).

In certain embodiments the vaccine comprises outer membrane vesicles obtained from one or more *H. ducreyi* strains. *H. ducreyi* causes genital ulcers in humans. An advantageous example of a *H*. *ducreyi* strain is the sequenced strain 35000HP (Fusco et al., 2010, Infect Immun 78:3763-3772), of which data obtained in human studies are available.

In certain embodiments the vaccine comprises outer membrane vesicles obtained from one or more *H. influenzae* strains and one or more *H. ducreyi* strains. In specific embodiments the vaccine comprises outer membrane vesicles obtained from one or more encapsulated *H. influenzae* strains and one or more nontypeable strains of *H. influenzae* (NTHi) and one or more *H. ducreyi* strains. These embodiments are particularly intended for human use.

In certain embodiments the vaccine comprises outer membrane vesicles obtained from one or more *P. multocida* strains and/or one or more M. *haemolytica* strains and/or one or more *Haemophilus somnus (H. somnus*) strains. In the first instance, these embodiments are intended for veterinary use, e.g. providing protection against bovine respiratory disease (BRD) and avian cholera. Non-exhaustive examples are sequenced strains (e.g. *P. multocida* Pm70 and M. *haemolytica* BAA-410), clinical isolates obtained from infected animals (e.g. *P. multocida* Pm70 or P4881 and M. *haemolytica* BAA-410). (Rimler, 1996, J Comp Pathol 114:347-360; May et al., 2001, PNAS 89:3460-3465; Gioia et al., 2006 J Bacteriol 188:7257-7266). H. *somnus,* also referred to as *Histophilus somni (H. somni),* is a common animal pathogen, mainly in cattle. An advantageous example of a H. *somnus* strain is H. *somnus* 2336 (Sandal et al., 2007, J Bacteriol 189:8179-8185). In specific embodiments the vaccine comprises outer membrane vesicles obtained from one or more *P. multocida* strains and one or more M. *haemolytica* strains and one or more H. *somnus* strains.

In another aspect, a method of inducing an immune response in a subject comprising administering to the subject an immunologically effective amount of OMVs in form of a vaccine according to the invention is disclosed herein. In one embodiment of the invention, the subject is a human being. In another embodiment, the subject is a domestic, farm or wild animal.

The vaccines according to the present invention may be administered to subjects at any appropriate therapeutically effective and safe dosage and dosage regime. The dosage and dosage regime may be determined by means of methods known by those skilled in the art, e.g. routine trials. The vaccines comprise an immunologically effective amount of OMVs obtained from only one *Pasteurellaceae* strain or an OMV-mixture comprising OMVs obtained from two or more *Pasteurellaceae* strains. The term "immunologically effective amount" means that the administration of that amount to an individual, either in a single immunization dose or as part of an immunization series, is effective for prevention against *Pasteurellaceae* infections. The immunologically effective amount depends on the subject to be immunized (e.g. human, taxonomic group of an animal), the desired level of protection, the subject's age, weight, sex, medical and physical condition, capacity of antibody production and other relevant parameters known to those skilled in the art. Dosage regimes may comprise one single immunization dose or multiple immunizations, e.g. a primary immunization followed by one or more booster doses.

The immunogenic response of a subject to a *Pasteurellaceae* vaccine according to the invention may be determined, for example, by measurement of antibody titers, lymphocyte proliferation assays, or by monitoring signs and symptoms after challenge with virulent wildtype strains. The protective immunity conferred by the vaccine can be determined by measuring the reduction in clinical parameters such as mortality, morbidity, and the physical condition and overall health of the subject.

Vaccines according to the invention are particularly suited to mucosal immunization. Routes of mucosal administration may include oral, intranasal, intragastric, pulmonary, vaginal, rectal, intestinal, and ocular routes. Intranasal or oral administration is preferred, and intranasal administration is particularly preferred. Moreover, it is also possible to administer the vaccine parenterally (e.g. intravenously, intramuscularly, intraperitoneally, subcutaneously, intracutaneously).

Where the vaccine is for intranasal administration, it may be in the form of a nasal spray, nasal drops, particle mists or a gel *etc.* Where the vaccine is for oral administration, it may be in the form of tablets, pills, capsules or troches, but also in liquid form such as tonics, syrups, suspensions, elixiers or drops *etc.*

Oral, subcutaneous, intramuscular or intranasal administration is particularly preferred in both humans and animals.

A "pharmaceutically acceptable diluent and/ or carrier" in the meaning of the present invention can be any substance used for the preparation of vaccines and which does not itself induce antibody production, including but not limited to coating materials, film-forming materials, fillers, disintegrating agents, release-modifying materials, carrier materials, diluents, binding agents, adjuvants and other substances used in formulating vaccines. Such carriers are well known to those of ordinary skill in the art. The vaccine according to the invention may comprise an adjuvant. Suitable adjuvants are known in the art, e.g. aluminium salts such as aluminium hydroxide and aluminium phosphate, oil formulations and emulsions. For example, adjuvant substances can be the B subunit of the cholera toxin, montanides, virus like particles and saponins (Reed et al., 2009, Trends Immunol 30:23-32).

In one aspect, a vaccine according to the invention comprises OMVs resuspended in a solution or buffer, e.g. phosphate-buffered saline (PBS), saline solution etc., and, preferably does not contain other components.

In a preferred embodiment, the vaccine according to the invention is free of an adjuvant. OMVs from *Pasteurellaceae* were shown to induce a robust immune response and a high level of protection in mice immunized with a vaccine according to the invention being free of an adjuvant.

A further aspect of the invention relates to a method for preparing a vaccine according to the invention comprising the steps of
(i) providing cells of one or more strains of the *Pasteurellaceae* family, with the proviso that hypervesiculating strains are excluded,
(ii) isolating outer membrane vesicles produced from these cells,
(iii) formulating a vaccine comprising the outer membrane vesicles as the only active components, wherein, in case of outer membrane vesicles from more than one strain, the outer membrane vesicles are mixed in a suitable ratio.

OMVs derived from *Pasteurellaceae* can be isolated by simple purifications steps. Upscale to industrial production has already been established for OMVs from N. *meningitidis* (Girard et al., 2006, Vaccine 24:4692-4700). In case that the vaccine comprises a mixture of OMVs obtained from more than one strain, the meaning of the term "suitable ratio" is as defined above. Protocols for isolating OMVs from *Pasteurellaceae* strains and formulating vaccines according to the invention are thoroughly described in the examples.

The present invention is further demonstrated and illustrated by the following examples, yet without being restricted thereto.

### EXAMPLE 1: Vaccines comprising OMVs obtained from one or more NTHi Strains

### Summary of the experiment

OMVs derived from NTHi strains give a high level of protection to challenge in experiments using mice. Specifically, female adult BALB/c mice were vaccinated via the intranasal route, boosted at two weeks and again at four weeks after initial immunization. The mice were categorized in three groups as follows: group 1 received immunization mix 1 (IM 1), group 2 received immunization mix 2 (IM 2), and group 3 served as an unvaccinated PBS-treated control group (naive). Immunization mix 1 (IM 1) consisted solely of OMVs derived from one NTHi strain, whereas immunization mix 2 (IM 2) contained a mixture of OMVs derived from three diverse NTHi strains reflecting the heterogeneity of NTHi strains as well as between members of the *Pasteurellaceae* family. No adjuvant was used for all immunizations. Serum antibodies to OMV antigens were analyzed by enzyme linked immunosorbent assay (ELISA) and immunoblot at the time of each boost for the immunized mice, as well as for unvaccinated naive mice. In general both immunization groups induced a robust immune response at comparable levels, whereas the naive mice showed no increase in immunoglobulin titers. Although the quantity of the induced immune response of both immunization groups was comparable, slight differences in the specificity could be observed by immunoblot analysis. The mice receiving IM 2 induced a more complex and multifarious immune response compared to the mice receiving IM 1. Ten days after the last boost naive and immunized mice were challenged for nasopharyngeal colonization using an intranasal inoculum of two different virulent NTHi strains. One NTHi strain was the donor strain for OMVs used in IM 1 and in part for IM 2, whereas the other NTHi strain was not used as a donor strain for OMVs in IM 1 or IM 2. Thus, for the immune system of the immunized mice the first one reflects a strain with "known" antigens, the second one reflects a strain with "foreign" antigens. All intranasal immunized mice were protected, i.e., depending on the NTHi strain used there was no or only low level colonization detectable having median values of 10 to 115 bacteria in their nasopharynx, respectively. In contrast, 100% of the unvaccinated mice were stably colonized having median values of 39.000 and 49.500 bacteria in their nasopharynx, respectively.

### Materials and Methods

### Bacterial Strains

Spontaneous streptomycin-resistant (Sm^{R}) derivatives 2019-R1, 3198-R1, 1479-R1, 9274-R3, and 7502-R1 of the respective NTHi strains 2019, 3198, 1479, 9274, and 7502 as well as 5657 were used as wild-type (wt) strains (Gu et al., 1996, Infect Immun 64:4047-53; Murphy and Apicella, 1985, Infect Immun 50:15-21). These are all clinical strains isolated from the sputum or middle ear of human patients. Sm^{R} derivatives were generated by plating over night (O/N) cultures of the respective strains on BHI agar supplemented with streptomycin. The use of Sm^{R} derivatives allowed the positive selection throughout the study including the challenge experiment. Each Sm^{R} derivative and respective donor strains were compared for their outer membrane and OMV protein profile and no obvious differences could be observed (data not shown). Furthermore *P. multocida* P4881 was isolated from a case of bovine pneumonia (Rimler, 1996, J Comp Pathol 114:347-60). Unless stated otherwise, bacteria were grown in Bacto™ Brain Heart Infusion (BHI, BD) broth or agar supplemented with NAD and hemin-solution (stocksolution containing mix of hemin, L-histidine, and triethanolamine) at 37°C with aeration. Supplements were used in the following final concentrations: NAD (Sigma) 10 µg/ml, hemin (Fluka) 20 µg/ml, L-histidine (Sigma) 20 µg/ml, triethanolamine (Merck) 0,08%, and streptomycin (Sm, Sigma) 100 µg/ml, respectively.

### Isolation of outer membrane (OM), outer membrane vesicles (OMVs), and whole cell lysates (WCL)

To isolate the OMVs produced from NTHi strains or *P. multocida* as well as their OM and WCL bacterial cultures (500 ml of BHI broth) were inoculated with 5 ml of BHI O/N stationary phase culture and grown to late exponential-phase for 13 h.

OM was isolated from 10 ml of the 500 ml culture as follows. Cells were harvested by centrifugation (4000 rpm, 10 min, 4°C) with an Eppendorf 5810R centrifuge and an A-4-81 rotor. The pellet was washed once in Hepes-buffer (10 mM, pH 7,4, Sigma) and finally resuspended in 1 ml Hepes-buffer with protease inhibitor (Roche, Complete EDTA-free protease inhibitor cocktail, 1 tablet per 50 ml). Cells were disrupted using sonification for 6 * 10 sec using a Branson sonifier 250. Unbroken cells were removed by centrifugation in an Eppendorf centrifuge 5415R (2 min, 13000 rpm, 4°C) and the supernatant containing the OM proteins was transferred into a new tube and centrifuged again (30 min, 13000 rpm, 4°C). The pellet was resuspended in 0.8 ml Hepes-buffer with 1% sarcosyl and incubated for 30 min. After centrifugation (30 min, 13000 rpm, 4°C), the pellet was washed once with 0.5 ml Hepes-buffer and finally resuspended in 50 µl Hepes-buffer.

In parallel WCL were prepared by sonification similar to the isolation of the OM as described above. After the removal of unbroken cells by centrifugation the remaining supernatant served as the WCL.

OMVs were isolated from the residual 500 ml culture. Cells were pelleted by two subsequent centrifugation steps (6000 rpm, 10 min, 4°C and 9500 rpm, 6 min, 4°C) using a Beckman- Coulter Avanti J-26 XP centrifuge and a JA-10 rotor. The supernatant was filtered consecutively through 0.45 µm and 0.2 µm pore size filters (Nalgene, 156-4045 and 156-4020) to give complete removal of remaining bacteria. To confirm the absence of viable bacteria, 200 µl of the filtrate was plated on a BHI agar plate, incubated for 48 h at 37°C and examined for colonies. No colonies were observed. The filtrate was stored at 4°C. Within the next two days OMVs were purified from the filtrate by ultracentrifugation (4 h, 29000 rpm, 4°C) using a Beckman-Coulter Optima L-100 XP ultracentrifuge and a SW32Ti rotor and resuspended in approximately 150 µl phosphate-buffered saline (PBS).

The protein concentration of OM, OMVs and WCL were determined by photometric measurements of the absorbances at 260 nm and 280 nm using a Beckman-Coulter DU730 spectrophotometer in combination with a TrayCell (Hellma) and the Warburg-Christian equation. The OMV solution was adjusted to 2.5 µg/ µl using PBS and stored at -70°C.

### Comparison of OM and OMV proteins

In order to determine if the protein content of the OMVs reflect in part that of the OM, we compared the protein profiles of the OM and OMVs from NTHi strains and *P. multocida.* OM proteins or OMVs were isolated as described above. A volume equivalent to 4 µg of each OMV or OM sample were mixed with 2 µl 5 x Laemmli buffer (55 mg/ml SDS, 20,5 mg/ml EDTA, 8,5 mg/ml NaH₂PO₄ x 2 H₂O, 92,5 mg/ml DTT, 25% glycerol, 0,1% bromphenolblue, pH 7,2), adjusted to 10 µl using PBS in case of OMVs or Hepes in case of OM, respectively. Samples were subsequently boiled for 10 min and finally loaded on a SDS-PAGE gel (12%) and electrophoretically separated (Mini-Protean Tetra System, Biorad). The prestained broad range protein marker (NEB) served as a molecular weight standard. Protein bands were visualized with Kang staining solution (0.02% Coomassie blue G-250, 5% aluminum-sulfate,10% EtOH, 2% phosphoric acid).

### Stability of OMVs

To test the stability of proteins within purified OMVs we incubated 15 µl of OMVs at -70, +25 or +37°C. After 9 days, a volume equivalent to 4 µg of each sample was mixed with 2 µl 5 x Laemmli buffer, adjusted to 10 µl using PBS, boiled for 10 min and finally loaded on a SDS-PAGE gel and electrophoretically separated (Mini-PROTEAN Tetra System, Biorad). Protein bands were visualized with Kang staining solution.

### Immunization with OMVs

BALB/c mice (Charles River Laboratories, Sulzfeld Germany) were used in all experiments. Mice were anesthetized by inhalation of 2.5% isoflurane gas prior to all immunizations. 9-week-old female mice were immunized at days 0, 14 and 28 with OMVs via the intranasal (i.n.) route using 25 µg in 10 µl PBS (5 µl per nostril) for all immunizations. In contrast, nonvaccinated control mice (naïve) received just PBS alone and were housed in parallel with the vaccinated mice for the duration of the experiment. Blood was collected by lateral tail vein nick at days 0, 14, and 28 as well as on day 39 by cardiac puncture. Additionally fecal pellets were collected on day 39.

### Serum and fecal pellet extract and preparation

To obtain the serum the collected blood was allowed to clot at room temperature for 30 min after which serum was isolated by removing the blood clod by centrifugation in an Eppendorf centrifuge 5415D (10 min, 4500 rpm). The supernatant was removed and diluted 4-fold in PBS. After adding sodium azide to a final concentration of 0.02% the serum was stored at - 70°C.

Three to five freshly voided fecal pellets of the respective mouse were vacuum-dried for 10 min using a Savant SpeedVac Concentrator before their weight was recorded. Igs were extracted by adding 1 ml of extraction buffer (PBS, 0.01% sodium azide, 5% fetal calf serum, 1 tablet Complete EDTA-free protease inhibitor cocktail (Roche) per 10 ml) per 100 mg feces and vortexing the samples for 15 min at 4°C. Solid material was separated by centrifugation (2 min, 13.000 rpm, 4°C) and the supernatants were stored at -70°C.

### Analysis of the quantity of the induced immune response by ELISA

IgA, IgG1, and IgM isotype antibodies to OMVs derived from 2019-R1 and 3198-R1 were determined by ELISA using 96-well ELISA Microplates (BD Falcon) essentially as described previously (Schild et al., 2009, Infect Immun 77:472-84; Schild et al., 2008, Infect Immun 76:4554-63). Plates were coated by incubation with OMVs (5 µg/ml in PBS) at 4°C O/N. To generate standard curves for each isotype, plates were coated in triplicate with 2-fold dilutions of the appropriate purified mouse Ig isotype standard (IgA, IgG1, or IgM, BD Biosciences) starting at 0.25 µg/ml in PBS. After washing four times with 0.05% Tween-20 in PBS (PBS-T), nonspecific binding sites were blocked with 10% heat-inactivated fetal calf serum (Invitrogen) in PBS (PBS-F) for 1 h at RT. Appropriate 5-fold dilutions of the test samples, starting at 1:100 (fecal pellet extract) or 1:400 (sera) in PBS-F were applied on the OMV coated wells in triplicate, whereas PBS-F was used for the wells coated with isotype standards.

Plates were incubated for 1 h at RT and washed four times with PBS-T. The plates were then incubated for 1 h at RT with the appropriate horseradish peroxidase-conjugated affinity purified goat antibodies against mouse IgA (α-chain specific, Southern Biotech), IgG1 (γ1-chain specific, Southern Biotech), or IgM (µ-chain specific, Southern Biotech). After four washes with PBS-T, plates were developed using the TMB Peroxidase Substrate (Biolegend) and 1M H₃PO₄ as stop solution according to the manufacturer's instructions. Optical densities were read at 450 nm with a Fluostar Omega plate reader (BMG Labtech). Titers were calculated using values from the appropriate dilutions of test samples and a log-log regression calculated from at least four dilutions of the isotype standards.

Half-maximum total Ig titers (IgA, IgG and IgM) to OMVs derived from 2019-R1, 3198-R1, 5657, and 7502-R1 as well as *P. multocida* P4881 were determined by ELISA as described above with the exception that horseradish peroxidase-conjugated affinity-purified goat antibodies against mouse (IgM + IgG + IgA, H+L, Southern Biotech) served as a secondary antibody, no Ig standard was used, and at least four five-fold dilutions starting at 1:100 were used to calculate the titers. Half-maximum titers were calculated using the solution of the sigmoidal line of the plot of the log of the reciprocal dilutions of mouse sera and the resulting absorbances to determine the reciprocal that gave half of the maximum optical density.

### Analysis of the quality of the induced immune response by immunoblot

The antibody response of the vaccinated and naive mice was analyzed by immunoblot using the TE 22 Mighty Small Transphor Electrophoresis Unit (Amersham Biosciences). SDS-PAGE with OMV and OM samples was performed as described above and subsequently proteins were transferred onto a Hybond™-C nitrocellulose membrane (Amersham Biosciences) using CAPS-buffer (10 mM CAPS, 10% methanol, pH 11). After the transfer the membrane was washed twice for 10 min in TBS (20 ml 1 M Tris pH 7.5, 30 ml 5 M NaCl in 1 liter) before the membrane was blocked by incubation in 10% milk powder (Roth) in TBS for 2 h at RT. For use as the primary antibody the mouse serum was diluted 1:500 in 10% milk powder in TBS. The diluted serum was added to a membrane and incubated at 4°C O/N on a rocker. The membrane was washed twice in TBS-TT (20 ml 1 M Tris pH 7.5, 100 ml 5 M NaCl, 2 ml Triton, 500 µl Tween-20 in 1 liter) and once in TBS for 10 min each. After washing, the membrane was incubated for 1 h in the secondary antibody solution using horseradish peroxidase conjugated anti-mouse IgG from goat (Dianova) in 10% milk powder in TBS. The membrane was washed four times in TBS-TT and once in TBS for 10 min each wash. Chemiluminescent detection was performed by using the Immun-Star™ WesternC™ Kit (Biorad) and exposure in a ChemiDoc XRS system (Biorad) in combination with Quantity One software.

### Analysis of the protective induced immune response by challenge

To first determine the infectious dose necessary to obtain a stable nasopharyngeal colonization the respective NTHi strains (2019-R1 or 3198-R1) were streaked for pure colonies on BHI agar plates and grown overnight at 37°C. Approximately 60 colonies were resuspended in BHI broth and grown O/N. On the next day this preculture was backdiluted in 3 ml fresh BHI broth a final optical density at 490 nm (OD₄₉₀) of 0.1 and grown for approximately 4 to 5 h to OD₄₉₀=1. Cells were harvested using Eppendorf centrifuge 5415D (5 min, 5000 rpm), resuspended and adjusted to an OD490 of 1.1 (equivalent to approximately 1 x 10⁹ CFU/ml) in PBS.

Subsequently 1:10 dilutions in PBS were prepared. The first 1:10 dilution was mixed 1:1 with PBS to obtain the infection mix (approximately 5 x 10⁷ CFU/ml). In parallel appropriate dilutions were plated on BHI plates supplemented with streptomycin and incubated for two days at 37°C to determine the CFU/ml by back calculating to the original suspension and the infection mix.

Mice were anesthetized by inhalation of 2.5% isoflurane gas and i.n. inoculated with approximately 5 x 10⁵ CFU using 10 µl (5 µl per nostril) of the infection mix.

After 24 h the mice were sacrificed by humane measures consistent with recommendations of for Euthanasia prepared for the European Commission DGXI (Close et al., 1996, Recommendations for euthanasia of experimental animals: Part 1. DGXI of the European Commission. Lab Anim 30:293-316; Close et al., 1997, Recommendations for euthanasia of experimental animals: Part 2. DGXT of the European Commission. Lab Anim 31:1-32) and the corresponding animal protocol (39/158 ex 2000/10), which has been approved by the "Bundesministerium für Wissenschaft und For-schung" Ref II/ 10b. The nasopharynx from each mouse was removed by dissection and mechanically homogenized (Tissue-Tearor, Biospec). Appropriate 1:10 dilutions of the homogenized nasopharynx were made in BHI broth, and plated for colony counts on BHI plus streptomycin plates. All mice were housed with food and water ad libitum and under the care of full time staff and in accordance with the rules of the department at the host institutions.

### Figures Legends

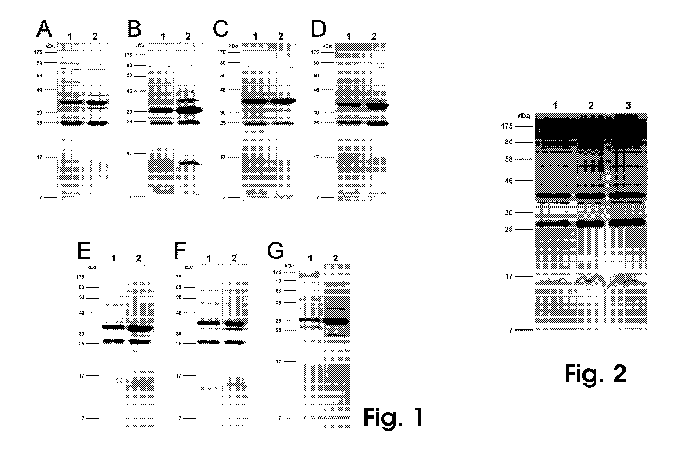

**Fig. 1****: Members of the *Pasteurellaceae* produce OMVs.** Comparison of OMV (lane 1) and OM (lane 2) protein profiles from NTHi strains 2019-R1 (A), 3198-R1 (B), 1479-R1 (C), 9274-R3 (D), 5657 (E), and 7502-R1 (F) as well as *P. multocida* strain P4881 (G). Samples were separated by SDS-PAGE and stained with Kang staining solution. Lines to the left indicate the molecular weights of the protein standards in kDa.
**Fig. 2****: Stability of OMVs.** Lanes 1, 2 and 3 show protein from OMVs derived from 2019-R1 stored for 9 days at -70°C, 25°C and 37°C, respectively. Samples were separated by SDS-PAGE and stained with Kang staining solution. Lines to the left indicate the molecular weights of the protein standards in kDa.

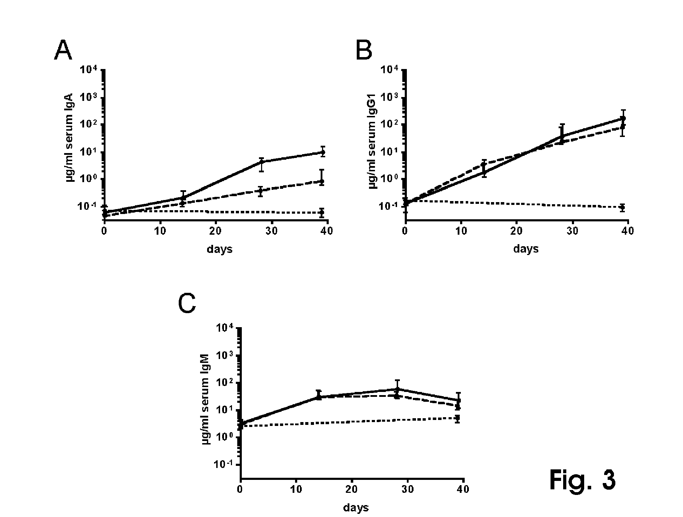

**Fig. 3****: Immunoglobulin titers to OMVs derived from NTHi strain 2019-R1 (present in IM 1 and IM 2).** Shown are the median titers over time of IgA (A), IgG1 (B), IgM (C) antibodies to OMVs in sera from mice i.n. immunized with IM 1 (solid line) and IM 2 (dashed line) as well as the PBS treated naive mice (dotted line) (n = 20 for each group). The error bars indicate the interquartile range of each data set for each time point.

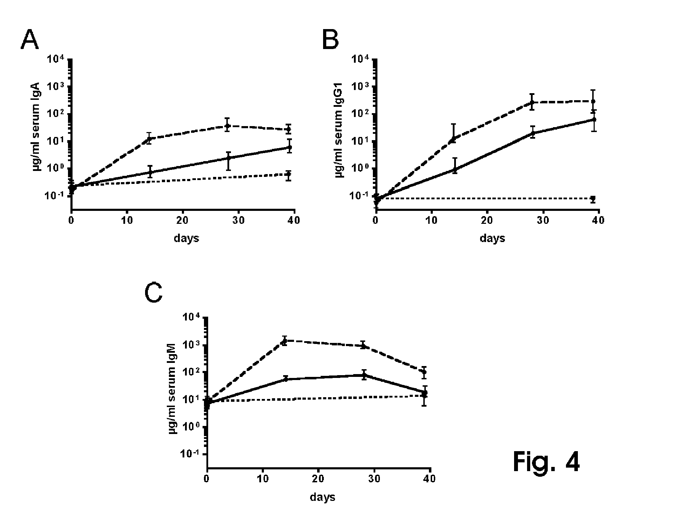

**Fig. 4****: Immunoglobulin titers to OMVs derived from NTHi strain 3198-R1 (not present in IM 1 and IM 2).** Shown are the median titers over time of IgA (A), IgG1 (B), IgM (C) antibodies to OMVs in sera from mice i.n. immunized with IM 1 (solid line) and IM 2 (dashed line) as well as the PBS treated naive mice (dotted line) (n = 20 for each group). The error bars indicate the interquartile range of each data set for each time point.

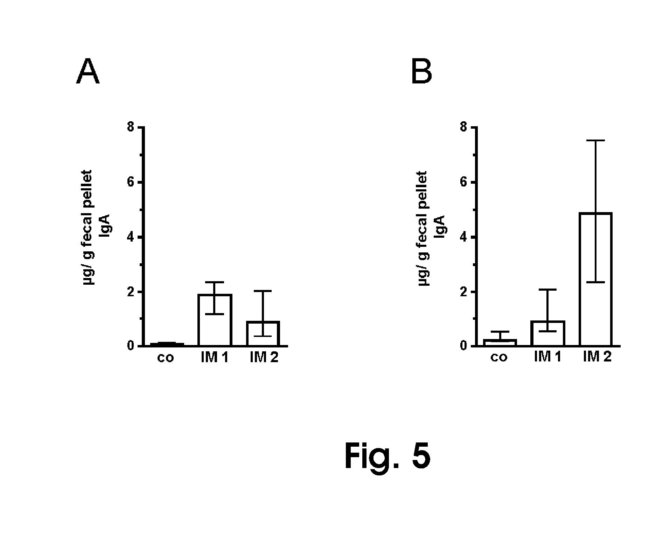

Fig. 5: Immunoglobulin titers in fecal pellets of mice **to OMVs derived from NTHi strain 2019-R1 and 3198-R1.** Shown are the median titers of IgA antibodies to OMVs derived from NTHi strain 2019-R1 (A) and 3198-R1 (B) extracted from fecal pellets collected on day 39 from mice i.n. immunized with IM 1 and IM 2 as well as the PBS treated naïve mice (control, co) (n = 10 for each group). The error bars indicate the interquartile range of each data set.

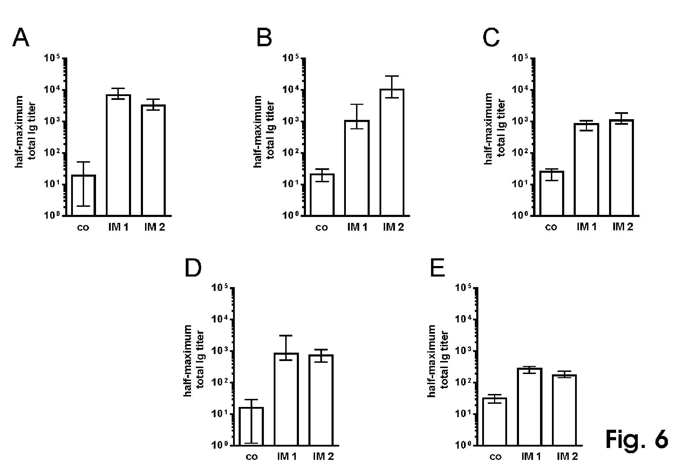

**Fig. 6****: Half-maximum total immunoglobulin titers in serum of mice to OMVs derived from several NTHi strains and** *P*. *multocida.* Shown are the median half-maximum total immunoglobulin titers to NTHI strains 2019-R1 (A), 3198-R1 (B), 5657 (C), and 7502-R1 (D), as well as *P*. *multocida* P4881 (E) for serum collected at day 39 from mice i.n. immunized with IM 1 and IM 2 as well as the PBS treated naive mice (control, co) (n = 20 for each group). The error bars indicate the interquartile range of each data set.

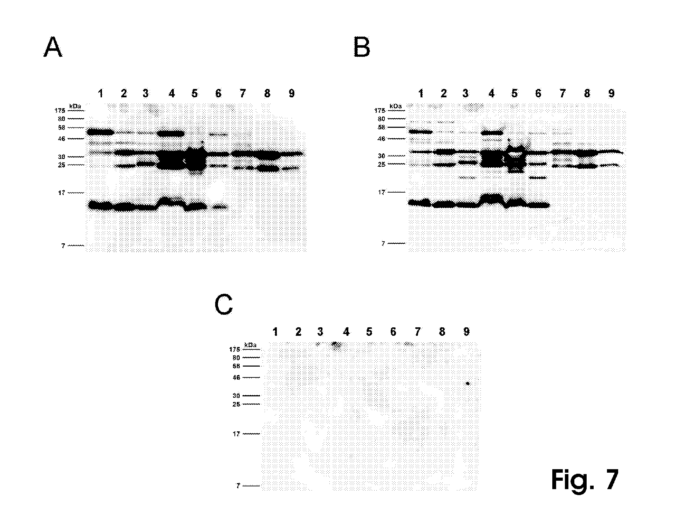

**Fig. 7****: Immunoblot analysis of IgG reactivity in sera from immunized mice.** Shown are representative immunoblots incubated with sera collected at day 39 from mice i.n. immunized with IM 1 (A) and IM 2 (B) and the PBS-treated naive mice (C). All immunoblots were loaded as follows: OMVs of 2019-R1 (1), OM of 2019-R1 (2), and WCL of 2019-R1 (3); OMVs of 3198-R1 (4), OM of 3198-R1 (5), and WCL of 3198-R1 (6); OMVs of *P. multocida* P4881 (7), OM of *P. multocida* P4881 (8), and WCL of *P*. *multocida* P4881 (9). Lines to the left indicate the molecular weights of the protein standards in kDa.

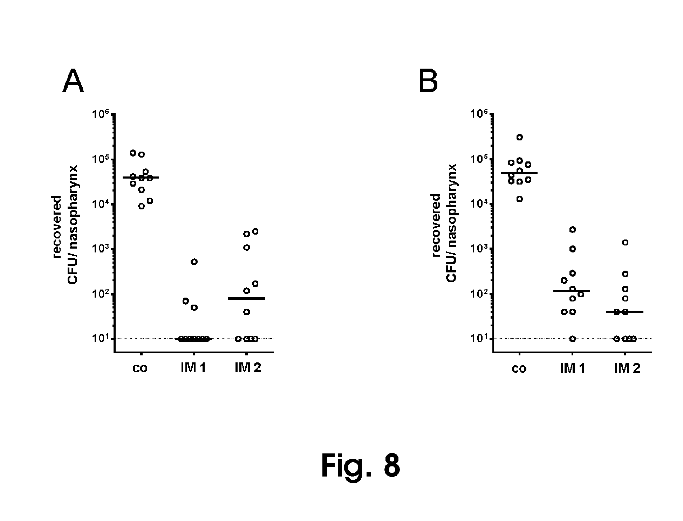

**Fig. 8****: Induced immune response of mice immunized with OMVs derived from NTHi is protective against nasopharyngeal challenge.** Shown are the recovered CFU per nasopharynx for mice immunized with IM 1 and IM 2 as well as the PBS treated naive mice (control, co) challenged with NTHi strains 2019-R1 (A) or 3198-R1 (B). Each circle represents the recovered CFU of one mouse. The horizontal bars indicate the median of each data set. If no bacteria could be recovered then the CFUs were set to the limit of detection of 10 CFU/nasopharynx (indicated by the dotted line). The CFU of the infection doses ranged from 3.3 x 10⁵ to 6.0 x 10⁵ CFU/ mouse for NTHi strain 2019-R1 and 4.1 x 10⁵ to 4.3 x 10⁵ CFU/ mouse for NTHi strain 3189-R1.

### Results

The results of the protein profile analysis of OMVs and OM derived from different NTHi strains as well as *P. multocida* P4881 are shown in Fig. 1. A direct comparison at the protein level of purified OMVs and the respective OM isolated from the same strain revealed similar patterns for all strains analyzed. This indicates that the abundant proteins of the OM are also present in the OMVs. Some bands are over- or underrepresented in the OMVs compared to the OM. Such enrichment or exclusion has been observed before for other bacteria, suggesting a sorting mechanism for at least some proteins (Kuehn and Kesty, 2005, Genes Dev 19:2645-55; Mashburn-Warren and Whiteley, 2006, Mol Microbiol 61:839-46). Furthermore OMVs also contain periplasmic proteins along with proteins of the outer membrane. Thus, it is not surprising to detect more proteins in the OMVs compared to the OM. In summary all strains tested release significant amounts of OMVs into the culture supernatant. These OMVs can be isolated using the aforementioned established protocol by centrifugation and filtration.

To test the stability of proteins within purified OMVs the inventors incubated OMVs at -70, +25 or +37°C. Again OMVs derived from strain 2019-R1 as representative for *Pasteurellaceae* OMVs were chosen. After 9 days, SDS-PAGE followed by visualization with Kang-staining was performed. As shown in Fig. 2 the proteins bands from all three samples are comparable in pattern and intensity indicating that the proteins were stable under all conditions tested for 9 days. No obvious degradation could be observed. Thus, isolated OMVs are very stable and a cold-chain is unlikely to be required for storage, shipping, and administration of a vaccine based on *Pasteurellaceae* OMVs.

Although the protein profiles of OMVs and OM derived from the same strain are quite similar, the comparison of the OMVs and OM protein profiles of the different NTHi strains revealed heterogeneity of the protein composition (Fig. 1). This diversity was also demonstrated by others and was used as a basis for a serotyping system of NTHi strains (Murphy and Apicella, 1985, Infect Immun 50:15-21). For the development of a broad-spectrum vaccine against several NTHi strains the observed antigenic heterogeneity is an open challenge. In the past the identification of ideal vaccine candidates focused on the search and use of conserved proteins present in the majority of NTHi strains. The approach in this invention is different. The inventive idea is to mimic the complexity of NTHi heterogeneity by presenting whole bacterial surfaces to the immune system. The inventors have chosen strain 2019-R1 to be the sole donor for OMVs used in immunization mix 1 (IM 1). This isolate is one of the best characterized NTHi strains with known LOS structure and has been extensively used in a variety of studies (Hirano et al., 2003, FEMS Immunol Med Microbiol 35:1-10; Lee et al., 1995, Infect Immun 63:818-24; Phillips et al., 1992, Biochemistry 31:4515-26; Tong et al., 2000, Infect Immun 68:4593-7). Additionally mixing OMVs derived from heterogenous NTHi strains can increase the antigen-complexity of the vaccine. Thus, the inventors have chosen three NTHi strains to be donors for OMVs used in immunization mix 2 (IM 2). As donor strains served strain 2019-R1, which was also used for IM 1, as well as strains 1479-R1 and 9274-R3. Purified OMVs from all three strains were mixed equally to prepare IM 2. In summary, IM 1 contained OMVs solely derived from NTHi strain 2019-R1, whereas IM 2 contained a mix of OMVs derived from 2019-R1, 1479-R1, and 9274-R3. The inventors immunized mice i.n. administration with IM 1 or IM 2 as described in Materials and Methods. A PBS-treated control group (naive) was housed in parallel for the duration of the experiment. Antibody titers in serum were monitored at four time points before (day 0), during (day 14 and 28) and after (day 39) the immunization period. The temporal IgA, IgG1, and IgM responses of each immunization group to OMVs derived from 2019-R1 or 3198-R1 are shown in Fig. 3 and 4, respectively. Since OMVs derived from 2019-R1 were present in IM 1 and IM 2, this reflects an immune response against "known" antigens in both immunization groups. In contrast neither the NTHi strain 3198-R1 nor its OMVs have been presented to the immunized mice, reflecting an immune response against "foreign" antigens. At day 0, the median isotype-specific antibody titers against OMVs derived from 2019-R1 and 3198-R1 were low levels comparable in all mice. Antibody titers in sera were monitored for the PBS-treated control mice (naive) at day 0 and 39, but the titers did not significantly change in this group during the entire experiment for all isotypes tested. For both immunization groups the median antibody titers of IgA and IgG1 increased during the vaccination period with highest level at day 28 or 39. No significant decrease of median IgA- or IgG1-titers has been observed for any immunization group. Median IgM-titers peaked on day 14 or 28 in all immunization groups followed by a slight decline most likely due to isotype switching. Mice immunized with IM 1 and IM 2 exhibited comparable immune responses for IgG1 and IgM to OMVs derived from 2019-R1 (Fig. 3B and C). Only IgA-titers to OMVs derived from 2019-R1 were significantly different between both immunization groups with higher levels in the IM 1 group compared to the IM 2 group on day 28 and 39 (Fig. 3A, p value <0.05 using a Mann Whitney U-test for both time points). In contrast, the induced levels of all three isotypes to OMVs derived from 3198-R1 were significantly higher in the IM 2 group compared to the IM 1 from day 14 to the end of the experiment (Fig. 4A-C, p value <0.05 using a Mann Whitney U-test for all time points).

Furthermore the induced mucosal immune response was investigated. Generally, the level of secreted antibodies to NTHi are analyzed from body fluids that are obtained either by performing nasal washes using PBS or collecting saliva after injection of pilocarpine to induce salivary secretion (Bertot et al., 2004, J Infect Dis 189:1304-12; Hirano et al., 2003, FEMS Immunol Med Microbiol 35:1-10). However, as it will become evident later, all mice were used for challenge experiments and i.n. inoculated with NTHi. Most likely nasal washes and injection of pilocarpine would have interfered with nasopharyngeal colonization. It has been shown before that IgA antibodies reflecting the mucosal immune response can also be found in feces and IgA levels in feces correlate with those in saliva (Hirano et al., 2006, Immunol Lett 107:131-9; Schild et al., 2009, Infect Immun 77:472-84; Vetvik et al., 1998, J Immunol Methods 215:163-72). Thus, in order to determine the induced mucosal immune response the inventors collected fecal pellets on day 39 from mice of both immunization groups and of PBS-treated control mice and extracted secreted antibodies. The determined IgA titers in fecal extracts from mice immunized with IM 1 and IM 2 as well as the PBS-treated control mice are shown in Fig. 5A and B. Only low levels of secreted IgA to OMVs derived from 2019-R1 and 3198-R1 could be detected in fecal samples of the control mice. Both immunization groups exhibited higher median fecal IgA titers to OMVs derived from 2019-R1 and 3198-R1 compared to the control mice. Highest median IgA titers to OMVs derived from 2019-R1 were detected in mice immunized with IM 1, whereas mice immunized with IM 2 exhibited highest median IgA titers to OMVs derived from 3198-R1. This tendency is consistent with the results of the isotype-specific antibody titers shown in Fig. 3 and 4.

Furthermore, the half-maximum total Ig titers in serum collected on day 39 from mice of both immunization groups and of PBS-treated control mice (Fig. 6) were determined. This allowed analysis of the induced immune response to OMVs derived from a variety of diverse NTHi strains (Fig. 6A to D) as well as *P. multocida* (Fig. 6 E). In all cases the halfmaximum total Ig titers of mice immunized with IM 1 or IM 2 were significantly higher than the PBS-treated control mice (p values <0.05 using a Kruskal-Wallis test and post-hoc Dunn's multiple comparisons). Depending on the origin of the OMVs used as antigens in the assay the level of the half-maximum total Ig titers of immunized mice ranged from 10-fold for *P. multocida* P4881 up to 1000-fold for 2019-R1 or 3189-R1 compared to the control group. As observed for the isotype-specific immune response the half-maximum total Ig titers to OMVs derived from 2019-R1 were slightly higher in mice immunized with IM 1, whereas mice immunized with IM 2 exhibited significantly higher titers to OMVs derived from 3198-R1. One should not forget that the total amount of OMVs derived from 2019-R1 in IM 2 was 3- fold less compared to IM 1. Thus, the almost identical half-maximum total Ig titers of mice immuniced with IM 1 and IM 2 to "known" antigens provided by OMVs derived from 2019-R1 clearly demonstrates that administration of an OMV mixture has no disadvantage for the recognition of antigens and induction of a immune response. Mice immunized with IM 1 or IM 2 had comparable half-maximum total Ig titers to OMVs derived from NTHi strains 5657 and 7502-R1 as well as *P. multocida* P4881.

In summary, the results of the ELISAs demonstrate the induction of a robust and complex humoral and mucosal immune response against members of the *Pasteurellaceae* in both immunization groups. Providing a complex mixture of OMVs derived from various isolates compared to OMVs derived from a single donor had no negative effect on the quantity of the induced immune response.

Immunoblots with OMVs, OM and WCL derived from NTHi strains 2019-R1 and 3198-R1 as well as *P. multocida* P4881 as antigen were used to test the specificity of the antibody response. Fig. 7 shows representative immunoblots utilizing sera collected on day 39 from one mouse immunized with IM 1 (Fig. 7 A), IM 2 (Fig. 7 B) or PBS-treated control group (Fig. 7 C). No bands were detected on the immunoblot using the serum from a PBS-treated control mouse, whereas the sera from immunized mice detected various bands in the OMV, OM and WCL protein profile of all strains tested. This clearly demonstrates that the OMVs used in IM 1 and IM 2 for immunization contained numerous proteins that can serve as antigens. In general the most reactive bands are located at approximately 50, 35, 25, and 15 kDa for NTHi as well as 35 and 25 kDa for *P*. *multocida.* The different specificity of the immune response induced in the mice immunized with IM 1 and IM 2 becomes obvious by comparison of the lanes loaded with OMVs from the different strains (Fig. 7A and B, lane 1, 4, and 7). These lanes reflect the surface exposed antigens of each strain. More bands are visible in panel B showing the immunoblot incubated with serum from a mouse immunized with IM 2 containing a mix of OMVs. This suggests a more complex immune response against a variety of different heterologous antigens in mice immunized with IM 2 compared to mice immunized with IM 1. Thus, a mixture of OMVs derived from different isolates providing a variety of heterogenous antigens is an advantage in the development of a broad-spectrum vaccine. Consistent with the results of the half-maximum total Ig titers the immunized mice of both immunization groups also induced an immune response against other members of the *Pasteurellaceae* family, e.g. *P. multocida.* Naturally the induced immune response against *P*. *multocida* is lower and less complex compared to the NTHi response, but this limitation can be overcome by including OMVs derived from *P. multocida* in the immunization mix.

In order to determine whether the immunization with OMVs was protective against colonization with NTHi, immunized mice were i.n. challenged and the level of protection was measured by the degree of colonization in nasopharynx after 24 h. Again the inventors used the 2019- R1 representing a strain with "known" antigens and the 3198-R1 representing a strain with "foreign" antigens. Previous experiments demonstrated that an infectious dose of ~ 10⁴ CFU of both NTHi strains already results in a stable colonization (data not shown). Mice immunized with IM 1 and IM 2 as well as the PBS-treated control mice were challenged with a 50-fold higher infection dose using approximately 5 x 10⁵ CFU per mouse. The results for the challenge with 2019-R1 and 3189-R1 are presented in Fig. 8A and 8B, respectively. All PBS-treated control mice were stable colonized with a median colonization at around 4 x 10⁴ CFU per nasopharynx. In contrast, i.n. immunized mice of both immunization groups exhibited significant protection shown by reduced colonization or no recoverable CFUs. In all cases the median colonization of immunized mice was below 1.2 x 10² and at least 300-fold lower compared to the control mice. Therefore, mice immunized with IM 1 and IM 2 induced a protective immune response to NTHi infection.

In summary, members of the *Pasteurellaceae* family release OMVs and OMVs derived from different isolates can be combined for immunization without disadvantages as demonstrated by the very effective immunization of mice receiving IM 2.

### EXAMPLE 2: Vaccine comprising OMVs obtained from P. multocida

### Materials and Methods

### Bacterial Strains

To further confirm the immunogenic potential of OMVs derived from members of the *Pasteurellaceae* family, a spontaneous streptomycin-resistant (Sm^{R}) derivative P4881-R1 of *P. multocida* strain P4881 (Rimler, 1996, J Comp Pathol 114:347-60; Ewers et al., 2006, Vet Microbiol, 114:304-317) is used. P4881 is a clinical strain isolated from a case of bovine pneumonia.

As described above in Example 1, the Sm^{R} derivative is generated by plating over night (O/N) cultures of the respective strains on Bacto™ Brain Heart Infusion (BHI, BD) agar supplemented with streptomycin and allows the positive selection throughout the study. The Sm^{R} derivative and the respective donor strain are compared for their outer membrane and OMV protein profile to confirm no obvious differences due to the generation of spontaneous streptomycin-resistant isolate (data not shown). Additionally, a M. *haemolytica* strain is used to determine the cross-reactive immune response (Gioia et al., 2006 J Bacteriol 188:7257-7266). *P. multocida* and M. *haemolytica* are grown in BHI broth or BHI agar supplemented with NAD (Sigma) 10 µg/ml and hemin (Fluka) 20 µg/ml at 37°C with aeration.

### Isolation of outer membrane (OM), outer membrane vesicles (OMVs), and whole cell lysates (WCL) as well as comparison of OM and OMV proteins

Isolation of OMVs, OM and WCL from M. *haemolytica* as well as their comparison using SDS-Page is performed as described in Example 1 for NTHi and *P. multocida* strains.

### Immunization with OMVs

Immunization studies using BALB/c mice (Charles River Laboratories, Sulzfeld Germany) are performed essentially as described in Example 1 for immunization with OMVs derived from NTHi strains. 9-week-old female mice were immunized at days 0, 14 and 28 with OMVs derived from *P. multocida* P4881-R1 via the intranasal (i.n.) route using 25 µg OMVs in 10 µl PBS (5 µl per nostril) for all immunizations. In contrast, non-vaccinated control mice (naïve) received just PBS alone and were housed in parallel with the vaccinated mice for the duration of the experiment. Blood was collected by lateral tail vein nick at days 0, 14, and 28 as well as on day 39 by cardiac puncture. Additionally fecal pellets were collected on day 39.

### Serum and fecal pellet extract preparation

Serum and fecal pellet are prepared as described in Example 1.

### Analysis of the quantity of the induced immune response by ELISA

IgA, IgG1, and IgM isotype antibodies to OMVs derived from *P. multocida* P4881-R1 and M. *haemolytica* are determined by ELISA using 96-well ELISA Microplates (BD Falcon) essentially as described in Example 1.

### Results

A direct comparison at the protein level of purified OMVs and the respective OM isolated from *P. multocida* P4881-R1 and M. *haemolytica* reveals similar patterns for both strains analyzed. Thus, both strains tested release significant amounts of OMVs into the culture supernatant and can be isolated using the aforementioned established protocol by centrifugation and filtration. To test whether the immunization with *P. multocida* OMVs also results in induction of a robust immune response, the inventors choose *P. multocida* P4881-R1 as donor for OMVs used in immunization mix IM 3.

Mice are immunized via i.n. administration using IM 3 as described in Materials and Methods. A PBS-treated control group (naive) is housed in parallel for the duration of the experiment. Antibody titers in serum are monitored at four time points before (day 0), during (day 14 and 28) and after (day 39) the immunization period. The temporal IgA, IgG1, and IgM responses of each immunization group to OMVs derived from *P. multocida* P4881-R1 and *M*. *haemolytica* are determined by ELISA. OMVs derived from *P. multocida* P4881-R1 are present in IM 3 and reflect an immune response against "known" antigens. In contrast, OMVs derived from M. *haemolytica* have not been presented to the immunized mice, reflecting an immune response against "foreign" antigens.

As already observed for the immunization experiment using OMVs derived from NTHi strains, median antibody titers of all isotypes increase during the vaccination period in the mice immunized with IM 3.

The induced mucosal immune response is determined from the collected fecal pellets on day 39 from immunized mice and of PBS-treated control mice. Furthermore, the inventors determine the half-maximum total Ig titers in serum collected on day 39 from these mice. Median fecal IgA titers and the half-maximum total Ig titers to OMVs derived from *P. multocida* P4881-R1 and *M*. *haemolytica* are higher compared to the control mice.

In summary, the results of the ELISAs confirm the induction of a robust and complex humoral and mucosal immune response against members of the *Pasteurellaceae* herein demonstrated for the genera *Pasteurella* and *Mannheimia.*

The data of the IM 3 experiment validate and strengthen the observed results obtained in the immunization study using NTHi derived OMVs and show the great potential for a broad-spectrum vaccine directed against a variety of members within the *Pasteurellaceae* family depending on the OMVs combined in the immunization mix.

## Claims

1. A vaccine comprising outer membrane vesicles as the only active components, wherein the outer membrane vesicles are obtained from one or more strains of the *Pasteurellaceae* family, with the proviso that hypervesiculating strains are excluded.

2. The vaccine according to claim 1, comprising outer membrane vesicles obtained from more than one strain of the *Pasteurellaceae* family.

3. The vaccine according to claim 1 or 2, wherein the vaccine comprises outer membrane vesicles obtained from one or more strains selected from the group consisting of wildtype strains, genetically modified strains, and combinations thereof.

4. The vaccine according to any one of claims 1 to 3, wherein the vaccine comprises outer membrane vesicles obtained from one or more strains of the genera *Haemophilus, Actinobacillus, Pasteurella, Mannheimia* or combinations thereof.

5. The vaccine according to claim 4, wherein the vaccine comprises outer membrane vesicles obtained from one or more H. *influenzae* strains.

6. The vaccine according to claim 5, wherein the one or more H. *influenzae* strains are nontypeable strains of H. *influenzae* (NTHi).

7. The vaccine according to claim 4, wherein the vaccine comprises outer membrane vesicles obtained from one or more *H*. *ducreyi* strains.

8. The vaccine according to claim 4, wherein the vaccine comprises outer membrane vesicles obtained from one or more H. *influenzae* strains and one or more H. *ducreyi* strains.

9. The vaccine according to claim 8, wherein the vaccine comprises outer membrane vesicles obtained from one or more encapsulated *H*. *influenzae* strains and one or more nontypeable strains of *H*. *influenzae* (NTHi) and one or more *H*. *ducreyi* strains.

10. The vaccine according to claim 4, wherein the vaccine comprises outer membrane vesicles obtained from one or more *P. multocida* strains and/or one or more M. *haemolytica* strains and/ or one or more *H*. *somnus* strains.

11. The vaccine according to claim 10, wherein the vaccine comprises outer membrane vesicles obtained from one or more *P. multocida* strains and one or more M. *haemolytica* strains and one or more *H*. *somnus* strains.

12. The vaccine according to any one of claims 1 to 11, comprising a pharmaceutically acceptable diluent and/ or carrier.

13. The vaccine according to any one of claims 1 to 12, wherein the vaccine is free of an adjuvant.

14. The vaccine according to any one of claims 1 to 13, wherein the vaccine is for intranasal, oral, subcutaneous and/ or intramuscular administration.

15. The vaccine according to any one of claims 1 to 14, for human and/or veterinary use.

16. A method for preparing a vaccine according to any one of claims 1 to 15 comprising the steps of
(i) providing cells of one or more strains of the *Pasteurellaceae* family, with the proviso that hypervesiculating strains are excluded,
(ii) isolating outer membrane vesicles produced from these cells,
(iii) formulating a vaccine comprising the outer membrane vesicles as the only active components, wherein, in case of outer membrane vesicles from more than one strain, the outer membrane vesicles are mixed in a suitable ratio.
